# EUROPEAN PATENT SPECIFICATION

(11) **EP 2 618 857 B1**
(45) Date of publication and mention of the grant of the patent: **22.10.2014**
(21) Application number: 11761103.8
(22) Date of filing: 16.09.2011
(51) Int. Cl.: A61L 27/56, A61B 17/11, A61L 27/18

(54) **MEDICAL DEVICE**
MEDIZINISCHE VORRICHTUNG
DISPOSITIF MÉDICAL

(30) Priority: 20.09.2010 GB 201015828
(43) Date of publication of application: 31.07.2013
(73) Proprietor: The University Of Manchester, Manchester M13 9PL (GB)
(72) Inventor: DOWNES, Sandra, Manchester M13 9PL (GB); SUN, Mingzhu, Bristol BS16 1JB (GB); MOBASSERI, Seyedeh, Atefeh, Manchester M13 9PL (GB); TERENGHI, Giorgio, Manchester M13 9PL (GB)
(74) Representative: Webster, Jeremy Mark
(86) International application number: PCT/GB2011/001358
(87) International publication number: WO 2012/038691

(56) References cited:
- WO-A1-01/81552
- Darice Y. Wong ET AL: "Brain cortex regeneration affected by scaffold architectures", Journal of Neurosurgery, 1 October 2008 (2008-10-01), pages 715-722, XP55014785, DOI: 10.3171/JNS/2008/109/10/0715) Retrieved from the Internet: URL:http://thejns.org/doi/pdf/10.3171/JNS/ 2008/109/10/0715 [retrieved on 2011-12-14]

## Description

### TECHNICAL FIELD OF THE INVENTION

The present invention relates to scaffolds for peripheral nerve repair, in particular to conduits through which peripheral nerves can grow as disclosed in claim 1. The present invention is also concerned with methods of making such scaffolds as disclosed in claim 14.

### BACKGROUND

The peripheral nervous system (PNS) extends outside the central nervous system (CNS) and provides the functions of, amongst other things, bringing sensory information to the CNS and receiving motor commands from the CNS, coordinating body movements and controlling the involuntary muscles. Unlike the central nervous system, the PNS is not protected by bone and is therefore vulnerable to injuries.

Damage to nerves of the PNS can cause significant motor or sensory impairment. In particular, patients with acute peripheral nerve injury usually have nerve conduction defects that can manifest as motor impairment or sensory dysfunction. Depending on the severity of the injury and the nerve affected, a severed nerve may cause paralysis, partial loss of mobility of the affected limb and/or a loss of sensation. Nerve and muscle atrophy will follow if no sufficient recovery occurs or no timely treatment is provided. Similarly, crush damage to peripheral nerves can result in reduced motor or sensory performance.

Surgical intervention is required if there is to be any prospect of repairing severed peripheral nerves. One surgical technique for attempting growth of a peripheral nerve involves providing a scaffold, usually in the form of a conduit, at the site of the nerve damage, to facilitate and encourage the extension of regenerating axons. Specifically, the scaffold is selected to provide an environment that will encourage nerve growth so that nerve function can be returned. To date, success rates for peripheral nerve growth have been low and it is presently not possible to achieve the extent of peripheral nerve growth that would be required in order to repair many of the injuries experienced by peripheral nerves. It has been suggested [1] that polyhydroxybutyrate (PHB) can be used to make peripheral nerve growth conduits, but, again, only low levels of peripheral nerve growth have been reported and the problem of repairing substantial peripheral nerve damage remains.

Furthermore, the direction in which axons grow along is an important issue and aberrant growth of axons results in neuromas.

The patent WO 01/81552 discloses a cylindrical substrate having a surface containing one or more substantially linear square-shaped grooves used for nerve regeneration.

### SUMMARY OF THE INVENTION

The present inventors have noted that in order for a peripheral nerve growth scaffold to effectively facilitate growth or repair of damaged peripheral nerves, it is desirable for the scaffold to exhibit a combination of properties as disclosed in claim 1.

Firstly, the present inventors have found that ordered, aligned or directed growth and proliferation of nerve and Schwann cells are important in order to optimise repair of a peripheral nerve and so the nerve conduit should provide an environment in which such behaviour can be achieved.

Secondly, the material from which the scaffold is made must be not only biocompatible but also subject to *in vivo* degradation at a rate which is sufficiently slow to ensure adequate time for the nerve to grow through the defect gap but fast enough to ensure that the scaffold does not remain at the site of the injury such that adequate healing can occur.

Thirdly, the present inventors have found that the mechanical properties of the scaffold must be such as to provide a robust and durable connection between the portions of the damaged peripheral nerve that is to be repaired (e.g. between proximal and distal stumps of a severed peripheral nerve), for example without breaking, swelling or collapsing once implanted. At the same time, the scaffold must exhibit sufficient flexibility to withstand handling and surgical implantation, as well as withstand movement experienced when *in situ.*

Fourthly, the present inventors have found that sufficient peripheral nerve growth is only likely to occur if the scaffold is a biocompatible substrate for nerve cells and Schwann cells. Suitably, the scaffold promotes or encourages the attachment and proliferation of peripheral nerve cells and Schwann cells; and it is desirable for the substrate to support the differentiation of nerve cells. The scaffold must therefore be non-toxic and should not release harmful break-down products. The scaffold should preferably also possess surface properties that mimic the basal lamina tissue *in vivo.*

Fifthly, the wall thickness of the nerve conduit should be small enough to avoid neuroma formation, rigidity and tissue compression associated with a thick wall. A thin wall, along with small device size, means less allogenic biological material and faster degradation rate.

At its most general, the present invention proposes that the above criteria are addressed by providing a peripheral nerve growth scaffold that comprises poly-ε-caprolactone (PCL) and a surface which comprises microgrooves adapted to encourage nerve and Schwann cell growth. This is based on the inventors' experiments wherein significant ordered nerve and Schwann cell growth occurred in a nerve conduit made from PCL having such microgrooves formed on the luminal (inner) surface of the conduit.

In a first aspect, the present invention provides a peripheral nerve growth scaffold including poly-ε-caprolactone (PCL) and microgrooves on an inner surface of the scaffold as disclosed in claim 1.

The present inventors have found that the combination of PCL and microgrooves, when provided as a scaffold, for example a conduit, surprisingly exhibits not only excellent mechanical properties and enhanced biocompatibility with peripheral nerve cells and Schwann cells but also provides a favourable environment for nerve and Schwann cell growth and proliferation. In particular the combination of PCL and microgrooves on an inner or luminal surface of the scaffold provides excellent bulk mechanical and biocompatible properties together with a surface environment adapted for directed growth and proliferation of nerve and Schwann cells. Such directed or aligned growth suitably reduces the rate of formation of neuromas.

Preferably the microgrooves form or are part of a pattern, for example an array of microgrooves. Thus, preferably the inner surface of the scaffold includes a pattern or array of microgrooves. Suitably the pattern or array comprises a plurality of microgrooves arranged side-by-side.

Suitably the microgrooves are aligned along the lengthwise or "long" direction of the scaffold. Thus, suitably, when the scaffold is inserted *in vivo* so as to bridge a gap in a peripheral nerve the microgrooves are aligned between the respective ends of the damaged nerve. In other words, it is preferred that the microgrooves are aligned in the direction of intended nerve growth. In the case of a tubular conduit scaffold, the microgrooves are preferably aligned substantially parallel to the longitudinal axis of the conduit.

Preferably the microgrooves extend to one or both ends of the scaffold, preferably both ends (the scaffold ends in use being associated with respective ends of the damaged nerve). For example this could be achieved with two sets of microgrooves, each set being associated with one of the scaffold ends, or a single set extending from end to end. Thus, suitably at least some microgrooves are substantially coterminous with the scaffold ends.

Preferably the microgrooves extend along at least 50% of the length of the scaffold, more preferably at least 60%, more preferably at least 70%, more preferably at least 80%, more preferably at least 90% and most preferably substantially all of the length of the scaffold.

Preferably the microgrooves are provided on at least 50% of the inner surface, by area, of the scaffold (e.g. conduit). That is, suitably the pattern comprising the microgrooves (i.e. the microgrooves themselves and the ridges or spaces between the microgrooves) is provided on at least 50% of the inner surface of the scaffold. More preferably the microgrooves are provided on at least 60% of the inner surface of the scaffold, more preferably at least 70%, more preferably at least 80%, more preferably at least 90%, more preferably at least 95% and more preferably substantially all of the inner surface.

The width of the microgrooves and the spacing of the microgrooves (i.e. the ridge between neighbouring microgrooves) can be selected independently. The width and the spacing can be the same or different. Preferably the width and the spacing are different. Suitably the width is larger than the spacing. Preferably the width is at least 1.5 times the spacing, more preferably at least 1.75 times the spacing, more preferably at least 1.9 times the spacing, more preferably at least 2 times the spacing, more preferably at least 2.5 times the spacing, more preferably at least 3 times the spacing, more preferably at least 3.5 times the spacing, more preferably at least 3.75 times the spacing and most preferably at least about 4 times the spacing.

Whilst there is no particular upper limit, a maximum ratio of 10:1, preferably 8:1, preferably about 6:1, more preferably about 5:1 and most preferably about 4.5:1 or 4:1 is preferred.

A particularly preferred range is about (width:spacing) 1.5:1 to 5:1, more preferably about 2:1 to 5:1, more preferably about 2.5:1 to 5:1, more preferably about 3:1 to 5:1, and most preferably about 3.5:1 to 4.5:1.

Preferably the microgrooves are substantially continuous, i.e. unbroken. Nevertheless, in embodiments some breaks or discontinuity may occur without a substantial adverse affect on the performance of the scaffold.

Suitably the width of the microgrooves is at least 2 µm, preferably at least 4 µm, more preferably at least about 5 µm, more preferably at least 8µm, more preferably at least about 10 µm, more preferably at least 13 µm, more preferably at least 15 µm, more preferably at least 18 µm and most preferably at least about 20 µm. In embodiments, a groove width of about 20µm was found to provide excellent results. But larger widths are possible, for example up to 50µm.

Preferably the width of the microgrooves is 50µm or less, preferably 30 µm or less, more preferably 25 µm or less and most preferably 22 µm or less.

A particularly preferred range for the width of the microgrooves is 2 µm to 30 µm, more preferably 4 µm to 25 µm, more preferably 8 µm to 25 µm and most preferably about 10 µm to about 20 µm.

In embodiments the width is selected from 4 to 6 µm, 8 to 12 µm, 13 to 17 µm and 18 to 22 µm. Widths selected from about 5 µm, about 10 µm, about 15 µm and about 20 µm are particularly preferred.

Suitably the width of each microgroove is substantially the same. Thus, suitably each microgroove in the pattern or array has substantially the same width.

Suitably the spacing between the microgrooves (e.g. the ridge width between the microgrooves, measured from respective ridge edges) is at least 1 µm, preferably at least 2 µm, more preferably at least 3 µm, more preferably at least 4 µm and most preferably at least about 5 µm.

In other embodiments a smaller spacing between the microgrooves is preferred. In embodiments the spacing between the microgrooves is less than 5 µm, optionally less than 4 µm, optionally less than 3 µm, optionally less than 2 µm, optionally less than 1 µm. In some embodiments the spacing between the microgrooves is 0 µm (i.e. the ridge is V-shaped).

Preferably the spacing between the microgrooves is 30 µm or less, more preferably 25 µm or less, more preferably 20 µm or less, more preferably 15 µm or less, more preferably 10 µm or less and most preferably 8 µm or less.

Particularly preferred ranges for the spacing of the microgrooves are 2 µm to 30 µm, 2 µm to 25 µm, 2 µm to 20 µm, 2 µm to 15 µm, 2 µm to 10 µm and 4 µm to 8 µm.

In embodiments, the spacing is selected from 4 µm to 6 µm, 8 µm to 12 µm, 13 to 17 µm and 18 µm to 22 µm. Widths selected from about 5 µm, about 10 µm, about 15 µm and about 20 µm are particularly preferred.

In embodiments, the groove spacing can be larger for deeper grooves. For example, a preferred spacing:depth ratio is 1:1 to 2:1, more preferably 1.2:1 to 1.8:1. In particularly preferred embodiments, the spacing and depths are as follows: spacing about 5µm depth about 3µm; spacing about 8µm depth about 5µm; spacing about 10µm depth about 7µm; and spacing about 15µm depth about 10µm.

Suitably the spacing between each pair of microgrooves is substantially the same. Thus, suitably each spacing (e.g. ridge between microgrooves) in the pattern or array is substantially the same.

The depth of the microgrooves can be selected independently of the width and/or spacing of the microgrooves. However, in embodiments, as discussed above, the depth can be related to the spacing and/or width. For example, where etching is used to create the grooves (e.g. by etching a template), groove width may increase as a function of increasing depth.

Suitably the depth (as measured at the deepest point in the case of a microgroove that has a contoured or profiled cross-section) is at least 1 µm, preferably at least 2 µm, more preferably at least 3 µm, more preferably at least 4 µm and most preferably at least about 5 µm.

A suitable upper limit for the depth is 10 µm, preferably 8 µm.

A particularly preferred range for the depth is 4 to 6 µm, with about 5 µm being especially preferred.

A preferred width:depth ratio is in the range 0.5:1 to 0.8:1, preferably 0.65:1 to 0.75:1 and preferably about 0.7:1.

Suitably, the depth of each microgroove is substantially the same. Thus, suitably each microgroove in the pattern or array has substantially the same depth.

A particularly preferred width:spacing:depth ratio is in the range 1.5:1:0.5 to 5:1:1.5, with 2.5:1:0.6 to 4.5:1:0.6 being particularly preferred.

A particularly preferred selection of width, spacing and depths is as follows:
(width+spacing+depth) 5+10+3, 5+15+3, 5+20+3, 5+25+3, 5+30+3, 5+40+3 and 5+50+3; 8+10+5, 8+15+5, 8+20+5, 8+25+5, 8+30+5, 8+40+5 and 8+50+5; 10+15+7, 10+20+7, 10+25+7, 10+30+7, 10+40+7 and 10+50+7; and 15+20+10, 15+25+10, 15+30+10, 15+40+10 and 15+50+10.

The cross-section (taken perpendicular to the longitudinal axis of the grooves) or profile of the microgrooves can have any desired shape, for example rectangular (i.e. vertical sides and a flat bottom). Indeed, vertical side walls are preferred in some embodiments.

Nevertheless, the present inventors have found that a microgroove having sloping or non-vertical sides can give rise to particularly good cell growth performance, especially cell alignment. In particular, a V-shaped, especially a truncated V-shaped, cross-section is preferred. Another preferred topography is a V-shaped ridge, wherein the sloping sidewalls of adjacent grooves meant at a point, effectively giving a zero ridge width. Sloping sidewalls are referred to herein as SL-grooves, and the V-shaped ridge variant as SLV-grooves.

In the case of V-shaped or truncated V-shaped configurations both side walls has an angle to the vertical of at least 20°, more preferably at least 30°, more preferably at least 40°, more preferably at least 50° and most preferably about 55°. Both side walls are at an angle selected from the above. Suitably both side walls have the same angle.

In embodiments, one or both side walls has an angle to the vertical in the range of 30° to 50°. Suitably both side walls have an angle to the vertical in the range of 30° to 50°.

The microgrooves have non-vertical side walls, the width of the microgroove as referred to herein is the width at the top of the microgrooves, i.e. generally at the widest point. In such cases, the depth as referred to herein is the deepest point of the microgroove cross-section.

Suitably the microgrooves are straight or linear microgrooves. As noted above, preferably the microgrooves are aligned parallel to the longitudinal axis of the conduit. Suitably there is substantially no branching of the microgrooves. Suitably the microgrooves are discrete microgrooves in the sense that they do not merge or intersect with each other.

Preferably the microgrooves are parallel to each other.

Suitably, the scaffold includes at least 50wt% PCL, based on the total weight of the scaffold. Preferably the scaffold includes at least 60wt% PCL, more preferably at least 70wt%, more preferably at least 75wt% and most preferably about 80wt% PCL. In particularly preferred embodiments, the scaffold consists essentially, preferably consists, of PCL.

Suitably the inner surface on which the microgrooves are formed comprises PCL, preferably consists essentially, most preferably consists, of PCL.

The PCL as used herein can be PCL homopolymer or PCL copolymer.

If the PCL is present as a PCL copolymer, it is preferred that the PCL monomer is present in an amount of at least 50wt% of the copolymer, based on the total weight of the polymer. Preferably at least 60wt% of the copolymer is PCL monomer, more preferably at least 70wt%, more preferably at least 80wt%, and most preferably at least 90wt%.

The present inventors have found that the degradation rate and/or the peripheral nerve cell adhesion properties of the scaffold can be further improved if the scaffold also includes polylactic acid (PLA). Suitably, the PLA is provided as a mixture with the PCL. Alternatively, the PLA may be provided as a copolymer with PCL.

Suitably, if the PLA is provided as a copolymer, i.e. as PCL-PLA copolymer, PCL and PLA are the only comonomers. However, further comonomers can also be present.

It is preferred that the PLA is provided as a mixture (blend) with the PCL.

Preferably no more than 50wt% of the scaffold is PLA, more preferably no more than 40wt%, more preferably no more than 30wt% and more preferably no more than 25wt%. A particularly preferred concentration of PLA is about 20wt%. This has been found to provide a good balance of mechanical and cell adhesion properties.

In this connection, if the content of PLA is greater than 50wt%, it may be difficult or impossible to form a conduit by heat sealing (discussed below). Furthermore, if the content of PLA is greater than 50wt%, the material may be too quick to degrade *in vivo.* Furthermore, phase separation may occur.

Incorporation of PLA in combination with PCL may provide improved peripheral nerve cell viability and/or proliferation. Addition of PLA in the amounts described herein also adjusts (typically increases) the rate of biodegradation of the scaffold.

Preferably the weight ratio of PCL: PLA is in the range 20:1 to 1:1. More preferably the ratio is in the range 10:1 to 2:1, more preferably 7:1 to 2:1, more preferably 6:1 to 2:1 and most preferably 5:1 to 3:1. A particularly preferred ratio is about 4:1.

The term "PCLA" is used herein to denote a combination of PCL and PLA. PCLA can be a mixture (blend) of PCL and PLA, or a PCL-PLA copolymer.

Suitably the PCL has a number average molecular weight (Mn) in the range 10,000 to 200,000. Preferably the Mn is in the range 20,000 to 140,000, more preferably 40,000 to 120,000 and most preferably 60,000 to 100,000. A particularly preferred Mn is about 80,000. Suitably the PLA, if present, has a number average molecular weight (Mn) in the range 10,000 to 100,000. Preferably the Mn is in the range 10,000 to 80,000, more preferably 10,000 to 50,000 and most preferably 20,000 to 40,000. A particularly preferred Mn is about 30,000.

The scaffold is a conduit. The conduit provides a luminal space (i.e. the lumen, cavity or channel within the conduit) in which peripheral nerve cells can grow (e.g. regenerating nerve fibres can grow inside the conduit, suitably in the lengthwise direction of the conduit). Typically a conduit wall surrounds and defines the luminal space.

The conduit wall consists essentially, preferably consists, of PCL or a PCL-PLA mixture (blend).

The conduit is tubular. The conduit has tubular conduit walls. The tubular conduit walls surround and define a substantially cylindrical luminal space.

The conduit has a circular cross section.

Preferably the conduit is substantially straight. However, the conduit can also be bent or curved.

The thickness of the conduit walls is in the range 10µm to 300µm. Preferably the conduit walls have a thickness in the range 10µm to 200µm, more preferably 10µm to 100µm, more preferably 20µm to 100µm, more preferably 20µm to 80µm and most preferably 55µm to 65µm. A particularly preferred thickness is about 60µm.

In other embodiments the preferred thickness of the conduit walls is in the range 20µm to 200µm, and more preferably 60µm to 180µm.

The present inventors have found that a conduit wall thickness as described above provides a good balance between degradation time, mechanical strength and flexibility and it also surprisingly permits microgrooves of a depth as described herein to be formed on the inner surface of the conduit, for example by film casting as discussed herein, without damage to the wall (e.g. film) structure and without significantly changing the mechanical properties of the conduit.

Suitably the length of the scaffold, e.g. the conduit, is selected to be appropriate to the nerve damage that is to be repaired. For example, if the peripheral nerve damage comprises a severed peripheral nerve with 10mm of the peripheral nerve missing, then the length of the scaffold will be chosen so as to be sufficient to bridge the gap in the peripheral nerve. Typically, the conduit will be longer (e.g. 10% to 50% longer) than the gap.

Typically, the scaffold has a length in the range 5mm to 50mm, more preferably 5mm to 30mm, most preferably 5mm to 20mm. As discussed above, preferably the microgrooves extend along at least 50%, preferably substantially all of this length.

As with the length of the scaffold, the width (measured from the outer surface of the scaffold), e.g. the diameter, of the scaffold is selected so as to be appropriate to the peripheral nerve damage that is to be repaired. Suitable diameters are in the range 1 to 5mm.

Preferably the scaffold is made from a film comprising PCL. The present inventors have found that film formation can provide control over mechanical and cell adhesion properties. In particular, as discussed below, film formation on a (patterned) template permits efficient and low cost formation of microgrooves whilst retaining good mechanical properties. Surprisingly, in embodiments films can be cast directly on to suitable templates and removed without damage to the film. This is a valuable advantage because it permits rapid and efficient formation of patterned films and provides consistent microgroove patterns.

Typically the film is formed by solvent evaporation. That is, it is preferred that a film comprising PCL is formed by dissolving or dispersing the PCL in a solvent, casting the resultant solution or dispersion onto a surface and allowing the solvent to evaporate. Suitably the surface is the surface of a template as described herein.

The present inventors have found that halogenated solvents are particularly effective for film formation. Naturally, the solvent should suitably be a liquid at room temperature. In particular, halogenated hydrocarbon solvents have been found to work well, especially halogenated alkanes (haloalkanes), alkenes, benzene and toluene. Particularly preferred are halogenated C₁₋₁₀ alkanes and alkenes.

Chlorinated solvents are particularly preferred. Chloro-substituted C₁₋₄ alkanes, especially chloro-substituted methane, are especially preferred.

The most preferred solvents are dichloromethane (DCM) and chloroform. DCM is particularly preferred. The present inventors have found that DCM permits good control over the properties of the film. In particular, the present inventors have found that the surface morphology of the film is controllable with DCM such that cell adhesion, for example, can be enhanced as compared to other solvents.

These solvents have been found to be a good solvent for casting on a template: the pattern is accurately and reliably transferred to the film and the film can be readily removed from the template. The surface morphology of the scaffold is discussed below.

Suitably the solvent is heated, for example to a temperature in the range 40-60°C. This may assist in dissolving the PCL.

Preferably the concentration of the PCL in the solvent is in the range 1 to 10% (wt/vol), more preferably 1 to 5%, and most preferably 2 to 4%. A particularly preferred concentration is about 3%.

Preferably the film is cast onto a template, for example a silicon and/or silica (silicon dioxide) template. The template can be a patterned silicon wafer for example. Suitably the template is cleaned to remove impurities (e.g. degreased) prior to casting.

Suitably the film is allowed to dry in air. Optionally, air flow is provided to facilitate evaporation of the solvent. The present inventors have found that controlled evaporation of the solvent produces the most desirable surface properties. Suitably, the solvent is allowed to evaporate for at least 24 hours, preferably at least 48 hours. Preferably, film drying/solvent evaporation occurs at room temperature.

Typically, after solvent evaporation has been completed, the film is washed. Suitable washing agents include water, preferably distilled water.

Preferably the film is sterilised, for example sterilised using UV radiation, y radiation or 70% ethanol. Indeed, any suitable known technique for sterilising can be used. UV radiation is preferred.

The present inventors have found that the advantageous properties of a scaffold comprising PCL and microgrooves can be further improved by treating at least one surface comprising the microgrooves of the scaffold with an alkaline composition. Preferably this is achieved by treating the surface prior to formation of the scaffold. In embodiments, a film is treated with an alkaline composition prior to forming a conduit from the film.

Preferably treatment with an alkaline composition includes exposing the surface to an alkaline composition. A preferred alkaline composition includes hydroxide. Suitably the alkaline composition is an aqueous solution. A particularly preferred composition is aqueous NaOH.

The strength (and hence alkalinity) of the alkaline composition can be adjusted so as to provide the desired surface modifying effect. In the case of NaOH, a concentration in the range *1 N* to 20*N* is preferred, with 5*N* to 15*N* being particularly preferred, and 8*N* to 12*N* being yet more preferred. In embodiments, a concentration of 10*N* is used.

The duration of the treatment can similarly be adjusted to provide the desired surface modifying effect. However, a duration of 30 minutes to 3 hours is preferred, with 30 minutes to 2 hours being more preferred and 45 minutes to 90 minutes being even more preferred. In embodiments, the treatment time is about 60 minutes.

Suitably, the surface of the scaffold that is treated is the surface that, in use, is exposed to a peripheral nerve growing volume. In other words, preferably the surface is a surface to which it is desired that peripheral nerve cells adhere and/or proliferate. In the case of the scaffold being a conduit, the surface is preferably a luminal surface of the conduit (i.e. an inward facing surface).

Without wishing to be bound by theory, the present inventors believe that alkali treatment of the scaffold causes ester hydrolysis of the PCL. Suitably this causes formation of -COOH and/or -OH terminated PCL chains. Thus, ester hydrolysis suitably occurs as a result of alkali treatment. The present inventors believe that the presence of the hydrolysed ester (and in particular the -COOH and/or -OH moieties) may be, at least in part, responsible for the observed enhancement of cell adhesion and/or cell proliferation.

Furthermore, the present inventors have found that treatment with an alkaline composition can increase the hydrophilicity of the surface without significant adverse affect on the microgrooves. Suitably this in turn enhances the attachment of peripheral nerve cells. This increase in hydrophilicity is demonstrated by an increase in the wettability of the surface. SEM studies of the microgroove pattern shows that the microgrooves retain their desired shape and dimensions after alkaline treatment.

Preferably after treatment with an alkaline composition, the surface is washed. Suitably the washing step removes residual alkali. Suitably the washing step returns the pH of the surface to neutral. Preferably water (especially distilled water) is used to wash the surface.

Preferably the scaffold is provided as a conduit. Suitably the conduit is formed from a film. Suitably this is achieved by bringing two opposite edges of the film together, preferably by rolling the film up. Typically the film is rolled around a conduit forming member. Suitably this provides the desired dimensions (e.g. diameter) of the conduit. The conduit forming member can be a cannula or other suitably dimensioned structure (e.g. a mandrel). The present inventors have found that the microgrooves are not adversely affected by this fabrication technique.

In embodiments, the conduit is formed from more than one film. For example, a plurality of films may be rolled up to provide a laminate structure (e.g. a conduit wall comprising a plurality of layers of film). In such embodiments, only the innermost film need comprise the microgrooves.

Suitably the edges of the film are fixed together. Preferably this is achieved by heat sealing the film in its rolled up state. For example, the rolled up film (suitably on the conduit forming member) is heat sealed. Preferably heat sealing is achieved using a hot plate, but other heat sources could be used. Thus, the conduit is suitably formed by rolling up a film and heat sealing the edges of the film. Suitably heat sealing occurs at a temperature in the range 50-100°C, for example about 60°C. In practice, the heat sealing temperature is selected based on the melting temperature (Tm) of the material. Melting temperature can be measured by DSC, for example. Other fixing methods can also be used. However, heat sealing is preferred, not least because the present inventors have found that the surface morphology of the film, including the microgrooves is maintained after heat treatment. A further advantage of this approach is that no other potentially toxic materials (e.g. super glue) are introduced to this system by using the heat sealing method.

Suitably the microgrooved side of the film becomes the luminal or inner surface of the conduit.

The surface of the scaffold that in use is exposed to a peripheral nerve growth volume is referred to herein as the inner surface of the scaffold (e.g. the inner or luminal surface of a conduit).

Preferably the scaffold comprises a surface which in use is not exposed to a peripheral nerve growth volume. This is referred to herein as the outer surface of the scaffold (e.g. the outer surface of a conduit).

Suitably the outer surface is not provided with microgrooves. Indeed, suitably the outer surface is substantially smooth.

When the scaffold is a conduit, it is preferred that the conduit wall does not include any pores extending through the thickness of the wall (through holes). This arrangement has been found to provide advantages because it prevents the escape of the regenerating axons from the conduit. It may also prevent ingrowth of fibrous tissues which can lead to unwanted scarring. This may assist in providing a controlled environment within the conduit for nerve repair.

However, in embodiments a small number of such pores can be present, for example no more than 5% of the surface area comprises such pores. Preferably no more than 2% and more preferably no more than 1% of the surface area comprises such pores. Suitably, if such pores are present, they have a diameter not larger than 15µm. Preferably they have a diameter in the range 1-10µm. If present, these pores can assist in avoiding the building up of pressure resulting from fluid retention.

Preferably the film used to form the scaffold has a tensile strength of at least 1 MPa. Preferably the film used to form the scaffold has a Young's modulus of at least 20MPa. Preferably the film used to form the scaffold has a maximum strain of at least 1 mm/mm.

Preferably the scaffold is flexible. In embodiments, the present inventors have found that the PCL scaffold is highly flexible. This flexibility reduces or avoids irritation to surrounding tissues.

In particular, the present inventors have found that a scaffold comprising PCL and having microgrooves on an inner surface provides an excellent combination of mechanical properties, making it suitable for handling by a surgeon, whilst providing a surface environment adapted for nerve and Schwann cell growth and proliferation.

Preferably the scaffold is used to treat peripheral nerve damage.

Peripheral nerve damage can be a gap in a peripheral nerve, i.e. a severed peripheral nerve. Alternatively or additionally, peripheral nerve damage can be a partially severed peripheral nerve. Alternatively or additionally, peripheral nerve damage can be a crushed peripheral nerve.

Suitably the scaffold provides a microenvironment at the injured site with protecting and promoting effects for the regenerating peripheral nerve. For example, it can prevent the infiltration of fibroblasts and the escape of regenerating neurites; at the same time it can contain endogenous growth factors *in situ.* Therefore, the scaffold is suitable for treating crushed/damaged peripheral nerves as well as severed peripheral nerves.

In particular, the scaffold of the present invention can be used to treat neurapraxia (nerve nonfunction), axonotmesis (axon cutting), and neurotmesis (nerve cutting).

It is envisaged that the scaffold of the present invention is used to treat some or all of these types of peripheral nerve damage.

It particular, the scaffold is preferably used to treat acute peripheral nerve injury.

The peripheral nerve damages can occur as a result of accidental injury, disease or surgical procedures. For example, peripheral nerve damage can occur as a result of a cut to the hands or feet, crush injuries, organ transplant, tumour removal, congenital birth defects or previous attempts to repair peripheral nerves.

The scaffold of the present invention can be used to repair peripheral nerve damage wherever it occurs in the body. Examples of peripheral nerves that are most frequently damaged include: palmar digital nerves, median nerves, the ulnar nerve and the radial nerve. Further examples include the brachial plexus and musculocutaneous nerves. Yet further examples (in the lower limbs) include plantar digital nerve, peroneal and the sciatic nerve.

In embodiments, the scaffold is used to enclose the affected part of the peripheral nerve (i.e. the damaged .portion).

In other embodiments wherein the peripheral nerve damage includes a severed peripheral nerve such that there is a gap in the peripheral nerve, the scaffold is positioned so as to bridge the gap between the respective proximal and distal ends of the severed nerve. In preferred embodiments wherein the scaffold is a conduit, the conduit is positioned so as to provide a guide for peripheral nerve growth between the proximal and distal ends of the severed nerve.

The scaffold can be attached to the peripheral nerve by any means known to the skilled reader. Suitably the scaffold is attached using a suture. Suitably the suture provides attachment between the epineurium and the scaffold. Bioglue can also be used.

The scaffold can be used to treat peripheral nerve damage in an animal, including humans and non-humans. Treatment of humans is particularly preferred.

To assist in the treatment of peripheral nerve damage, the scaffold may be used in conjunction with a peripheral nerve cell growth medium (e.g. a gel matrix). For example, the peripheral nerve cell growth medium includes growth factors and Schwann cells. Suitably the peripheral nerve cell growth medium is a transport media for cells and/or growth factors (i.e. the peripheral nerve cell growth medium is, or comprises, a transport matrix). Typically the peripheral nerve cell growth medium is a hydrogel.

In use, the peripheral nerve cell growth medium (e.g. a gel matrix) is preferably introduced into the scaffold *in situ.* Typically the scaffold is positioned at the site of the peripheral nerve damage (e.g. after suturing) and then the growth medium (gel matrix) may be delivered to the scaffold. In preferred embodiments wherein the scaffold is a conduit, suitably the growth medium is delivered into the luminal volume of the conduit, optionally together with cells and/or growth factors. Suitably this is achieved by injecting the growth medium, for example through the end of the conduit after suturing.

While the invention has been discussed above in relation to a scaffold, the present disclosure also provides methods and uses relating to the scaffold.

In a further aspect, the present disclosure provides a peripheral nerve growth conduit, wherein the conduit includes poly-ε-caprolactone and microgrooves on a luminal (inner) surface of the conduit.

In a further aspect, the present disclosure provides a peripheral nerve growth conduit, wherein the conduit includes poly-ε-caprolactone and polylactic acid and microgrooves on a luminal (inner) surface of the conduit.

In a further aspect, the present disclosure provides a peripheral nerve growth conduit as described herein, wherein the conduit is prepared by solvent evaporation wherein the solvent comprises a halogenated solvent, preferably dichloromethane or chloroform.

In a further aspect, the present disclosure provides a peripheral nerve growth scaffold as described herein, wherein at least part of the surface of the scaffold has been treated with alkali.

In a further aspect, the present disclosure provides a peripheral nerve growth scaffold as described herein, wherein at least one surface of the scaffold includes -COOH and -OH groups.

In a further aspect, the present disclosure provides a peripheral nerve growth conduit as described herein, wherein the thickness of the wall of the conduit is in the range 20-100 µm. In a further aspect, the present disclosure provides a kit for treating a peripheral nerve in a human or animal, the kit including a peripheral nerve growth scaffold as described herein.

Preferably, the kit includes the peripheral nerve growth scaffold in a sterilised package.

Preferably, the kit includes a plurality of peripheral nerve growth scaffolds as described herein. More preferably, the peripheral nerve growth scaffolds vary in size according to one or more of the following dimensions: scaffold length, scaffold internal diameter and scaffold wall thickness. A user may then select the correct size of nerve repair scaffold from the kit to suit the requirements of a particular nerve repair treatment. Suitably, each peripheral nerve growth scaffold in the kit is in an individual sterilised package.

In a further aspect, the present disclosure provides use of poly-ε-caprolactone (PCL) in a peripheral nerve growth scaffold as described herein.

In a further aspect, the present disclosure provides use of hydroxide to treat the surface of a peripheral nerve growth scaffold as described herein. Suitably the action of the hydroxide encourages growth of peripheral nerves on said surface.

In a further aspect, the present disclosure provides use of PCL for the manufacture of a peripheral nerve growth scaffold as described herein for treatment of a damaged peripheral nerve.

In a further aspect, the present disclosure provides PCL for use in treating a damaged peripheral nerve using a peripheral nerve growth scaffold as described herein.

In a further aspect, the present disclosure provides a method of treating a damaged peripheral nerve using a peripheral nerve growth scaffold as described herein.

In a further aspect, the present disclosure provides a method of treating a severed peripheral nerve, the method including the steps of
(i) providing a peripheral nerve growth scaffold as described herein,
(ii) coupling a first severed end of the nerve to a first portion of the scaffold, and
(iii) coupling a second severed end of the nerve to a second portion of the scaffold,
wherein the first and second portions of the scaffold are separated by a growth portion of the scaffold having a growth surface comprising microgrooves on which at least one of the first and second severed ends of the nerve is able to grow in a direction towards the respective other severed end.

Suitably the first severed end is the proximal end of the nerve and the second severed end is the distal end of the severed nerve.

In a further aspect, the present invention provides a method of making a biocompatible polymer material having a patterned surface, the method comprising the steps of
(A) forming a negative of the desired pattern on a template, and
(B) applying the biocompatible polymer material to the template.

Suitably the pattern is a pattern of microgrooves as described herein.

Suitably the biocompatible polymer material is a biocompatible polymer, preferably a biodegradable polymer, preferably PCL. Suitable PCL-containing polymers are described herein. Preferably it consists essentially of and most preferably consists of PCL.

Suitably the method comprises the step of (C) removing the biocompatible material from the template. Preferably the biocompatible polymer material is treated with an alkaline composition, suitably as described herein.

Suitably the method comprises the step of (D) forming a nerve repair scaffold from the patterned biocompatible polymer material. Suitably the scaffold is formed so that the patterned surface is an inner surface of the scaffold, e.g. a luminal or inner surface of a conduit. Step (D) may comprise rolling up the patterned biocompatible polymer material. In embodiments the heat sealing method described herein is used to form the scaffold. Suitably the scaffold is sterilised.

Thus, preferably, the method is a method of making a nerve repair scaffold, suitably a peripheral nerve repair scaffold, more suitably a peripheral nerve repair scaffold as described herein.

Suitably step (B) includes forming a film of the biocompatible polymer material on the template. Suitable film thicknesses are as disclosed herein.

Preferably step (B) comprises (i) apply a solution or dispersion of the biocompatible polymer material (e.g. PCL) in a solvent to the template, and (ii) removing solvent from the solution or dispersion. In other words, a casting method to form a film is preferred.

Suitable solvents are described herein.

In embodiments, the solution is a solution of PCL. Suitably the solvent is a halogenated solvent as described herein, preferably DCM. The solution may contain other polymers, as discussed herein.

Preferably the biocompatible polymer solution (e.g. PCL solution) is applied directly to the template, i.e. without an intervening layer.

Typically the template comprises a substrate. Suitably the substrate is an inorganic material, preferably silicon. In embodiments the substrate is a silicon wafer.

Suitably the method comprises a photolithography process. Preferably the method comprises an etching process, preferably photolithography and etching processes.

Preferably step (A) includes forming a barrier layer on the surface of the substrate (e,g. a silicon wafer). Preferably the barrier layer comprises silicon dioxide. The barrier layer, e.g. silicon dioxide, can be thermally deposited onto the surface of the substrate, for example by heating the silicon substrate, for example at 1100 to 1200°C in a furnace.

Suitably a photoresist is formed on the substrate or a barrier layer, preferably on a barrier layer. Suitable photoresists are known to the skilled reader. Typically the photoresist is applied by spin coating. In embodiments the photoresist-coated substrate is heated, for example at about 110°C, to remove solvent.

A photomask is preferably used to demarcate the desired pattern. Typically the photoresist is exposed to radiation, typically UV radiation, through the photomask.

Suitably the solubilised areas of photoresist are removed, typically with a developer solution.

Preferably a post-developed heating step is carried out, for example to improve durability of the remaining photoresist.

Suitably the barrier layer (e.g. silicon dioxide) is etched, the pattern of etching being dictated by the pattern of the remaining photoresist. A preferred etching solution is HF (aq), preferably buffered, for example with NH₄F.

In embodiments, etching comprises plasma etching, for example Deep Reactive Ion Etching (DRIE).

After etching of the barrier layer preferably the remaining photoresist is removed by solvent washing, for example using acetone.

In embodiments the resultant material, comprising the substrate with etched (patterned) barrier layer, is then etched. That is, the substrate is selectively etched in areas where it is not covered by the barrier layer. A suitably etching material is KOH.

Suitably etching of the substrate is carried out for sufficient time to achieve the desired pattern depth in the substrate. Preferred dimensions for the microgrooves are discussed above.

The etched substrate is then typically washed with a solvent, suitably isopropanol.

Thus, embodiments of the present invention apply template-forming techniques from semiconductor chip manufacturing to biocompatible materials such as PCL to form microgroove patterns.

In a further aspect, the present invention provides a method of making a peripheral nerve repair conduit comprising PCL and having microgrooves on a luminal surface of the conduit, the method comprising the steps of
(A) forming a negative of the microgrooves in a template using photolithography and etching;
(B) applying a solution comprising PCL and a solvent to the template and removing the solvent to form a film comprising PCL;
(C) removing the film from the template; and
(D) forming the film into the conduit such that the microgrooves are on the luminal surface of the conduit.

Preferably the PCL solution is applied directly to the template, i.e. without an intervening layer.

In embodiments of the solvent casting method described herein, biocompatible polymers can be simply dissolved and poured onto a master (template) cured, and removed. Many replicates (copies) can be fabricated from a single patterned master; as such the cost and processing time can be minimized.

In a further aspect, the present invention provides a peripheral nerve growth scaffold including a biocompatible material and microgrooves on an inner surface of the scaffold, wherein the microgrooves have a width:spacing ratio in the range 1.5:1 to 8:1.

Preferably the biocompatible material is a biocompatible polymer, preferably a bioresorbable polymer. Particularly preferred is PCL.

Preferably the width:spacing ratio is in the range 2:1 to 5:1.

Preferably the width is in the range 10µm to 30µm, more preferably 18µm to 22µm, for example about 20µm.

Preferably the spacing is in the range 5µm to 15µm, more preferably 5µm to 10µm, more preferably 3µm to 7µm, for example about 5µm, about 8µm or about 10µm.

Suitably the depth of the microgrooves is in the range 3µm to 10µm, more preferably 3µm to 7µm, for example about 5µm.

Suitably the microgrooves are as described herein.

### BRIEF DESCRIPTION OF THE DRAWINGS

Embodiments of the invention and experiments illustrating the advantages and/or implementation of the invention are described below, by way of example only, with respect to the accompanying drawings, in which:

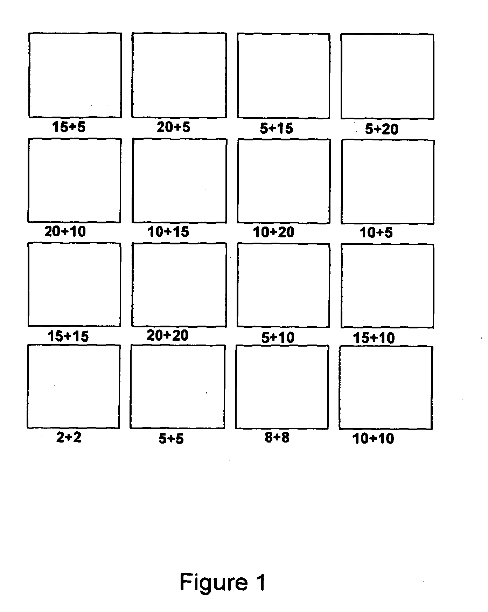

Figure 1 shows the design of a photo mask (Mask 1) for making patterned silicon wafer template using photolithography. Sixteen 1.3cm² square areas were included to test 16 different combinations of dimensions with alternating grooves and ridges. Dimensions of the pattern were displayed as ridge/spacing width plus groove width in micrometers;

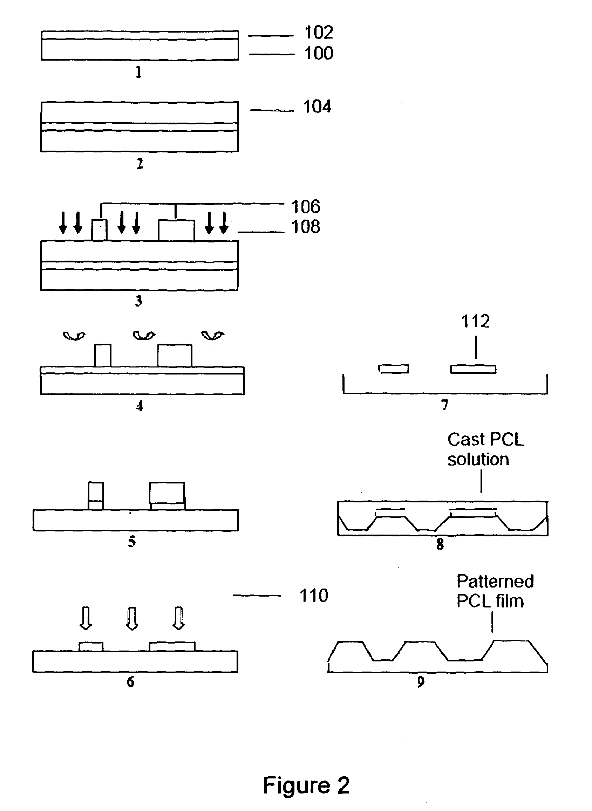

Figure 2 shows fabrication process for patterned PCL films: (1) A layer of SiO₂ (500nm) was grown on top of the silicon wafer; (2) A layer of photoresist was spun cast on the surface of a <100> silicon wafer; (3) Patterned photo mask was applied on top of the photoresist followed by UV exposure; (4) Pattern formed in developer solution; (5) HF etched through SiO₂; (6) Acetone washed off photoresist; (7) KOH etched through silicon wafer; (8) PCL solution was cast onto the master template; (9) Patterned PCL films peeled off the template;

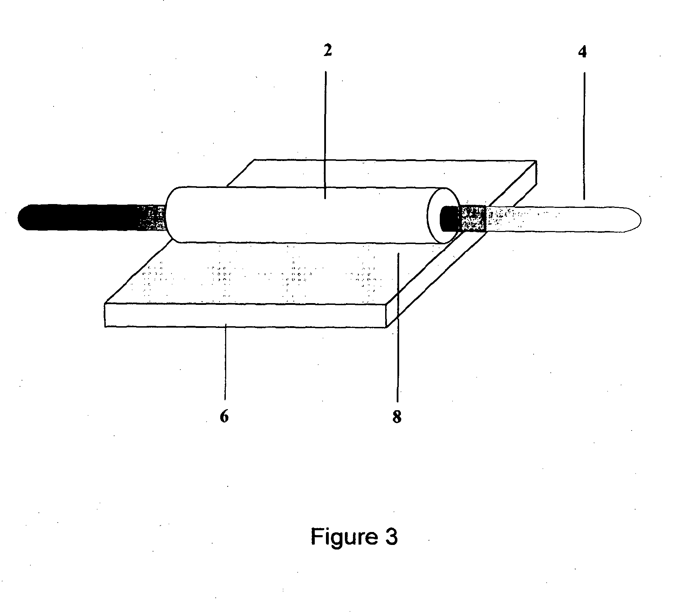

Figure 3 shows schematically the heat sealing method preferred for forming the conduit of the present invention;

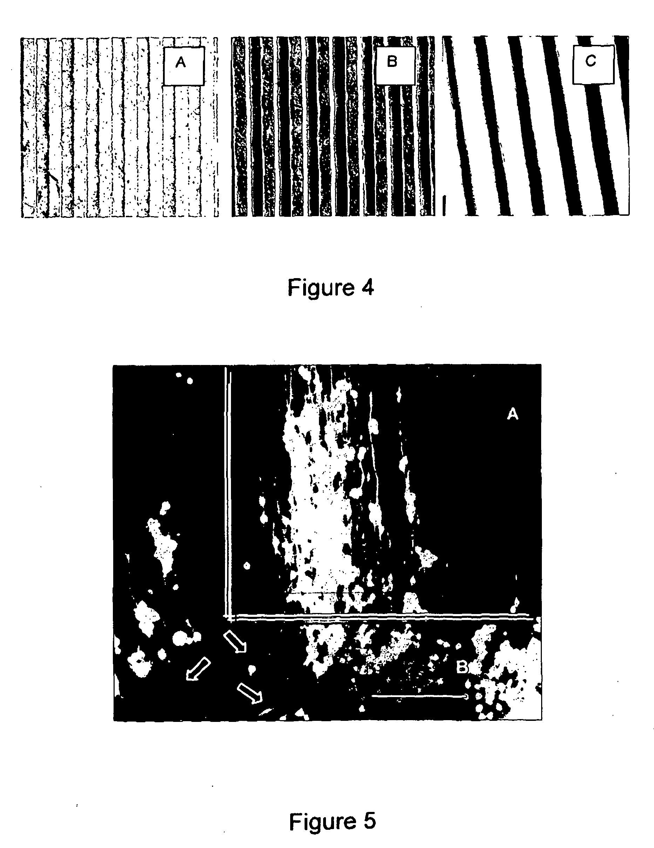

Figure 4 shows SEM (A and B) and AFM (C) images of patterned PCL films produced using photolithography and subsequent HF and KOH etching; the width of spacings and grooves shown in these pictures is 10 µm (Bar=40µm) for A and B and 8µm for C, depth of grooves is 5 µm for B and C, depth of grooves in A is 500 nm;
Figure 5 shows anti-neurofilament stained NG108-15 cells on PCL films having (A) patterned (8µm+8µm) and (B) un-patterned areas, showing contrast orientation effect of neurites in aligned and un-aligned areas; cells were differentiated for 7 days; arrows are pointing to randomly aligned nerve fibres in the non-patterned area (Bar=200µm);

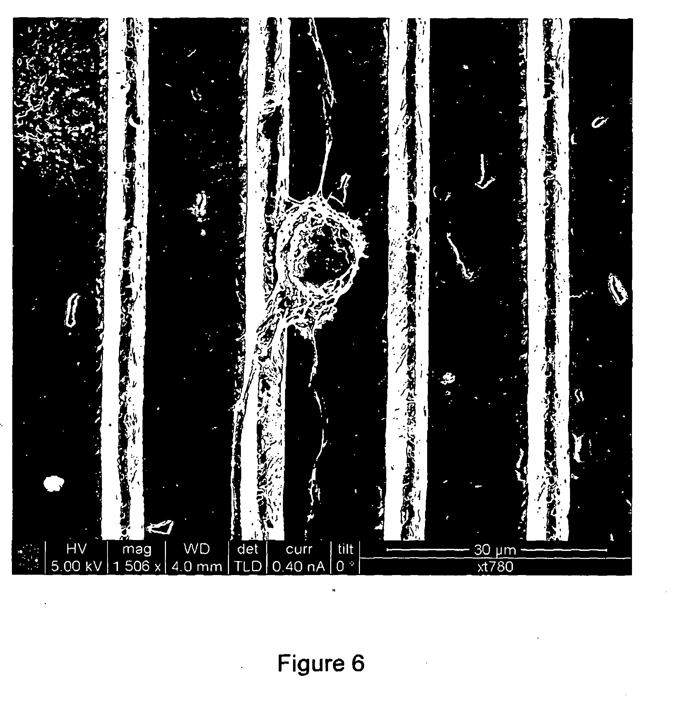

Figure 6 shows an SEM image of a differentiated NG108-15 cell aligned on patterned PCL film (5µm spacings and 15µm grooves (Bar=30µm);

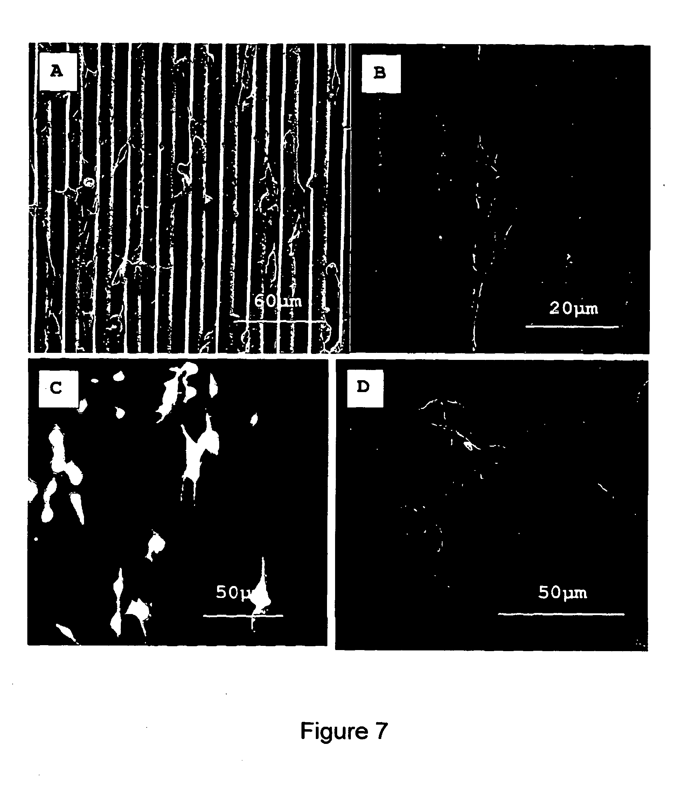

Figure 7 shows SEM images of Schwann cells on patterned PCL films (10µm + 10µm) after 5 day of culturing; (A) and (B) show aligned Schwann cells; (C) is immuno-stained Schwann cells using antibody against S 100 marker protein (green); nuclei of cells were counter-stained using DAPI (blue); (D) shows Schwann cells on unpatterned surface area with random alignment;

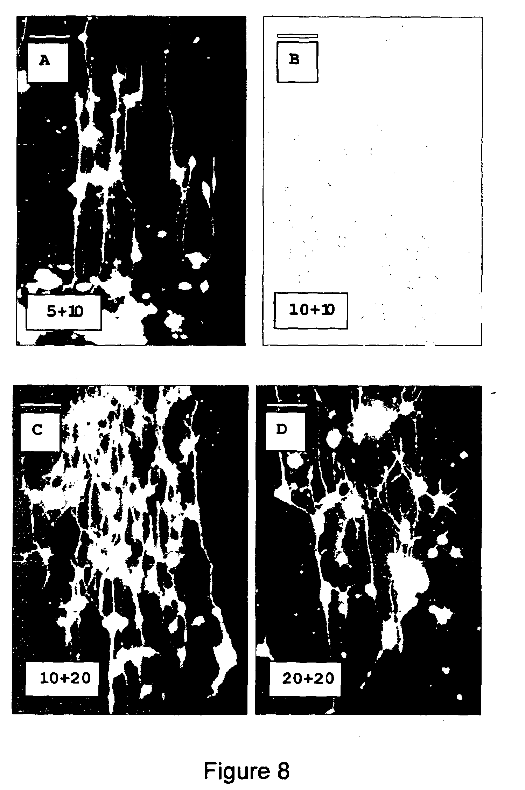

Figure 8 shows NG108-15 cells on different surface patterns: differentiated NG108-15 cells growing on patterns with wider grooves and spacings (C and D) tended to have more branched neurites than cells growing on patterns with small groove width (A and B) and the number of neurites also tended to be higher (Bar=20µm);

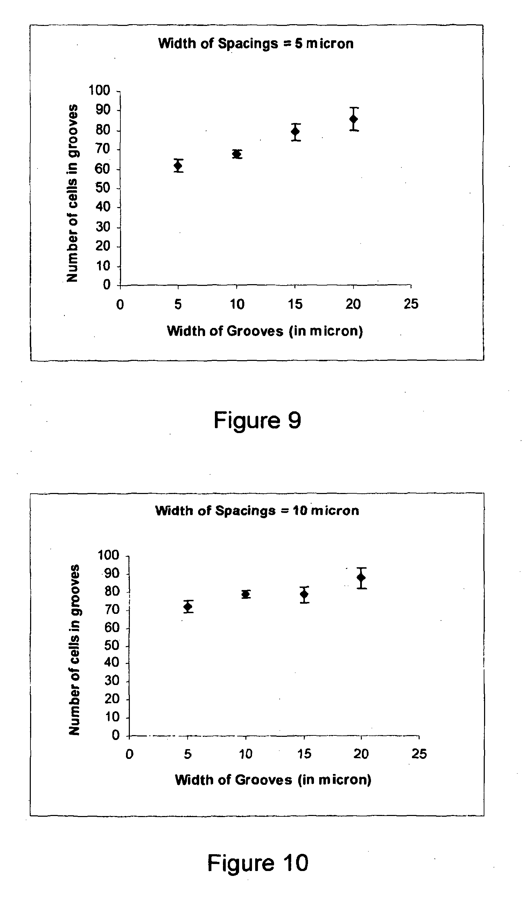

Figure 9 shows the number of NG108-15 cells growing in the grooves as a function of groove width at a spacing of 5µm;
Figure 10 shows the number of NG108-15 cells growing in the grooves as a function of groove width at a spacing of 10µm;

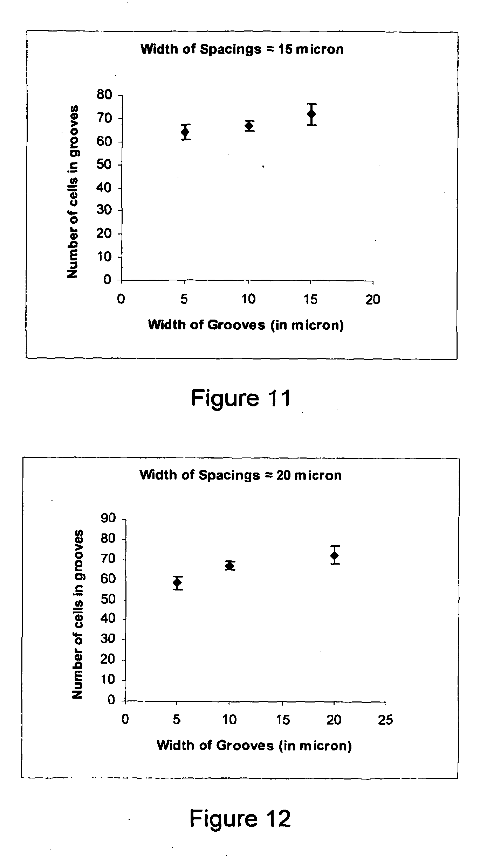

Figure 11 shows the number of NG108-15 cells growing in the grooves as a function of groove width at a spacing of 15µm;
Figure 12 shows the number of NG108-15 cells growing in the grooves as a function of groove width at a spacing of 20µm;

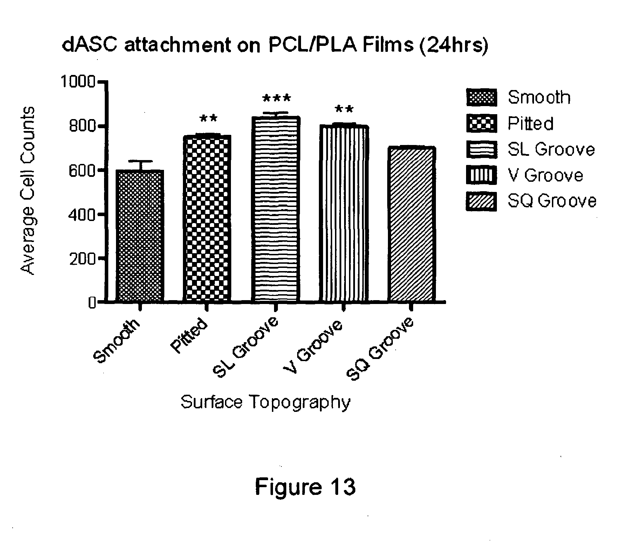

Figure 13 shows quantitative analysis of dASC attachment to PCL/PLA samples. The histogram represents differences in cell attachment of dASCs to PCL/PLA samples with different surface topographies after 24 hours. The data is expressed as mean cell counts ± SEM, n=3, **p* < 0.05, ***p* < 0.01 and ****p* < 0.001;

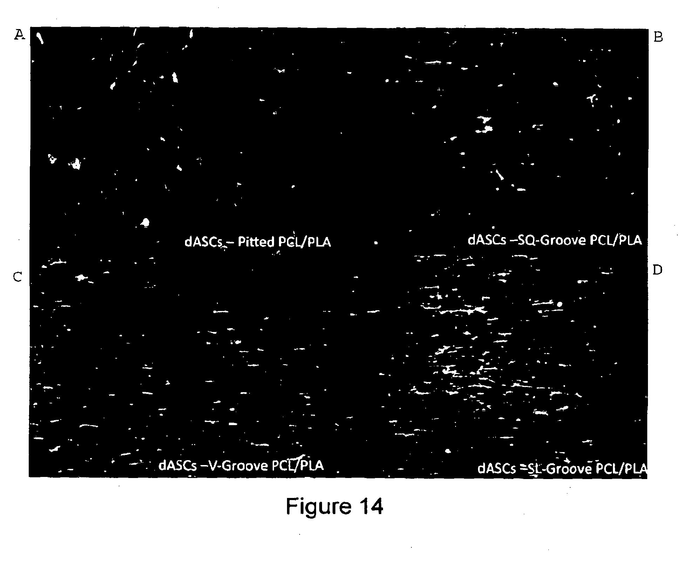

Figure 14 shows cell spreading and alignment on PCUPLA (4:1) pitted and grooved surfaces. Fluorescent images demonstrate that dASCs increase their cell attachment (DAPI stained blue nuclei) and alignment (phalloidin stained green actin) on SLV-grooved (C) and SL-grooved (D) surfaces when compared to smooth (A) and SQ-grooved surfaces (B). Images were taken using a 4x objective; and

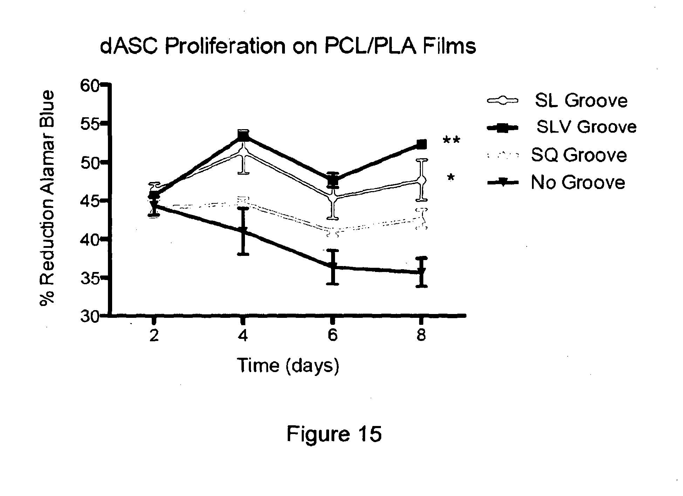

Figure 15 shows dASC proliferation or PCUPLA films, specifically alamarBlue® cytotoxicity/proliferation assay of dASCs on PCUPLA samples. The line graphs represent differences in dASC proliferation on to PCUPLA samples over an 8-day period. The data is expressed as mean percentage reduction of alamarBlue ± SEM, n=3, where **p* < 0.05 and ***p* < 0.01

### DETAILED DESCRIPTION OF THE INVENTION

### Definitions

The term "scaffold" as used herein is well known to the skilled reader. In particular, a scaffold in the context of the present invention is a structure adapted for peripheral nerve growth. Suitably the scaffold promotes or enhances peripheral nerve growth.

The term "microgroove" as used herein means a groove having a width of at least 1 µm. In this connection, the skilled reader will understand "groove" to include elongate channels and trenches formed on or in the surface of the scaffold material.

### Film Formation

PCL pellets (Sigma-Aldrich average *M*n ∼80,000g/mol) were dissolved in dichloromethane (3.0%, wt/v) and gentle heating at a temperature of approximately 50°C could be used to assist dissolving. PCL solution was evenly applied onto the desired template, which had been cleaned in Decon, dH₂O, and then isopropanol, followed by drying with an N₂ gun. If appropriate, the template can be vacuum dried before fabrication of polymer films.

Complete solvent evaporation was allowed in a fume cupboard for at least 48 hours, to provide films with a thickness of 60±5µm.

The polymer films were peeled away from the template, washed in distilled H₂O and sterilized by UV irradiation for 1 hour prior to testing.

Complete solvent evaporation was confirmed by FTIR (Thermo Nicolet Nexus^{™} FTIR (Cambridge, UK) controlled by OMNIC Software Version 6.1 a), which ensured that no solvent toxic effect would occur in the subsequent cell growth testing.

Using the same method, a mixture of PCL and PLA was formed as a film (the "PCLA film"). The weight ratio of PCL to PLA was 4:1.

### Pattern forming

Chrome photo mask was manufactured by Photronics UK Ltd.

### Design of photomask

Mask 1: A chrome photo mask was designed with 16 different areas, each comprising a microgroove pattern with different groove and spacing sizes (Figure 1). For example, "5+15" represented a 1.3 cm² square area with grooves 15µm in width and separated by spacings (ridges) 5 µm in width. All of the patterns were on a single substrate to limit sample variability and to conduct experiments in a high-throughput fashion.

Mask 2: Based on the results obtained from Mask 1, a new mask with 4 selected patterns (5+5; 10+10; 20+20, and 5+20) and enlarged pattern area (2.7x2.7cm²) was produced for fabricating implantable nerve conduits.

### Photolithography

To make the desired patterns, n-type silicon wafers (100) (4- inch in diameter with a <100> crystallographic orientation) were firstly cleaned to remove particulate matter from the surface as well as any other traces of impurities. The cleaning procedure used a mixture of sulphuric acid and hydrogen peroxide. Following cleaning, a barrier layer (approximately 500 nm in thickness) of silicon dioxide (102) was thermally deposited onto the surface of the substrate by placing the silicon wafer in an 1100-1200°C furnace for 40 mins (step (1) of Figure 2). After the formation of the SiO₂ layer, a thin uniform layer of photoresist (104) (Shipley 1813) was applied onto the wafer by means of spin-coating at 5000 rpm for 30 seconds (step (2) of Figure 2). The photoresist-coated wafer was then "prebaked" to drive off excess solvent at 110° for 20 mins. The photoresist was exposed through the photo mask (106) using standard contact-mode optical photolithography under UV light (108) for 50 seconds (step (3) of Figure 2). UV exposure changed the chemical property of the photoresist and rendered it more soluble in the developer solution (Shipley Microposit 351). A brief post-exposure bake was performed before developing. The exposed resist was then washed away by the developer solution (60 seconds) and an exact copy of the pattern remained on the wafer (step (4) of Figure 2). The resulting wafer was then "hard-baked" at 120° for 30 mins to solidify the remaining photoresist in order to make a more durable protecting layer for further processing.

### Etching of silicon wafer

Masked by photoresist, a solution referred to as buffered HF containing concentrated HF (49%) and a buffering salt (NH₄F) in the ratio of 1:4 was used to etch through the layer of SiO₂ (step (5) of Figure 2). Photoresist was removed by washing in acetone (110) (step 6 of Figure 2). Silicon wafer (100) was then anisotropically etched using 30% potassium hydroxide (KOH) (112) (step (7) of Figure 2). KOH solution was heated up to reach equilibrium at 80° C, which will etch silicon <100> planes at approximately 1.5 µm/min. Etching was conducted for a varying length of time to generate grooves in truncated V-shape at a depth of approximately 3µm or 5 µm. This is because the etching rate is also dependent on the width of the patterns-the wider the pattern, the faster the etching. The depth of the micro-grooves was monitored using a Form Talysurf-50 Profilometer (Taylor Hobson). SiO₂ was used as a mask/barrier because it is etched at a slower rate (1-2nm/min). A control template was made without KOH etching; it had the same pattern but with much shallower groove depth, being the depth of SiO₂ layer (approximately 500 nm).

The resulting template was washed in Decon, dH₂O and isopropanol and dried using a nitrogen gun. To form films, PCL solution (3% wt/v) was directly cast onto the surface of the patterned wafer (step (8) of Figure 2). A thin PCL film with the designed pattern could be easily peeled off the template (step (9) of Figure 2).

### Fabrication of silicon template by Deep Reactive Ion Etching (DRIE)

DRIE method was utilised to produce grooved silicon template with upright walls (i.e. substantially non-sloping sides). The desired pattern was produced on a silicon wafer by photolithography then the samples were etched using the DRIE process. In detail, silicon wafer (100) with silicon nitride barrier layer was cut in required dimension (2.7cm* 2.7cm). Then the sample was cleaned with acetone, IPA and oxygen plasma cleaning process. The photoresist, S1805, was then applied to the silicon substrate by spin coating. The photoresist-coated silicon was then heated to remove the remaining solvent. The substrate was exposed to UV radiation through the photomask. The developer, MF319, removed the exposed photo resist. Then the hard baking step was carried out to bake the photoresist on the substrate.

DRIE is a dry etching process to fabricate deep, steep-sided trenches in wafers, which uses reactive plasma to etch. Reactive particles diffuse to the silicon wafer and react and adsorb at the surface then the volatile reaction particles diffuse away from the surface. The main technique in DRIE is the Bosch process.

The Bosch process consists of passivation and etching cycles. At the passivation step, C4F8 based plasma is deposited to a few monolayers thickness across the silicon surface. Then in the etching step, SF6 is introduced to the system to isotopically etch the silicon. At the beginning of the etching step the ions bombard the surface and remove fluorocarbon from the surfaces parallel to the wafer surface. After selective etching, the unprotected silicon surface is exposed to reactive fluorine-based species and isotropically etched, whilst the vertical walls protected from etching by fluorocarbon polymer. By controlling the passivation and etch time during the process the degree of lateral etch is reduced. Furthermore, this process can lead to a smooth vertical wall in the wafer.

The resulting template was washed in Decon, dH₂O and isopropanol and dried using a nitrogen gun. To form films, PCL solution (3% wt/v) was directly cast onto the surface of the patterned wafer (step (8) of Figure 2). A thin PCL film with the designed pattern could be easily peeled off the template (step (9) of Figure 2).

### Alkaline (hydroxide) Treatment

PCL films were soaked in 10*N* NaOH for 1 hour with horizontal shaking at 150 rpm at room temperature and then rinsed thoroughly with distilled H₂O to return the pH to neutral (pH 7.2-7.4). Subsequent XPS analysis (discussed below) confirmed the cleavage of the ester bond (ester hydrolysis) as follows:

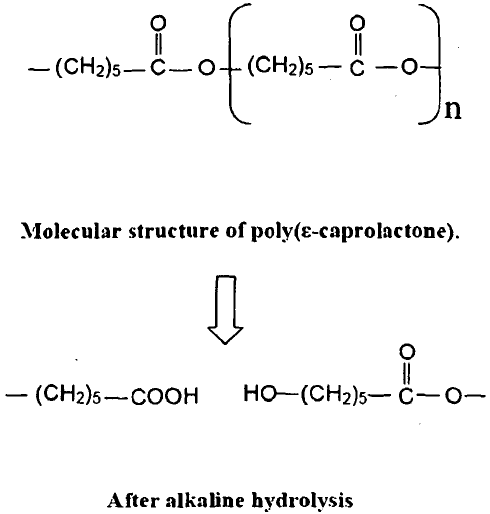

### Conduit Formation

Figure 3 illustrates schematically the methodology used to form the PCL conduits. The film 2 comprising the patterned surface destined to become the inner or luminal surface of the conduit, was wrapped around a 16G cannula 4, to form a tubular conduit. Sealing of the overlapping edges of the film was carried out by briefly (several seconds) pressing the edges on to a hot plate 6 at 60°C. A thin layer of tin foil was provided (at location 8) between the outer surface of the conduit and the hot plate. This provided a durable seal and the resultant tubular conduit was self supporting.

The inner (luminal) surface of the PCL conduits including the microgrooves was unchanged as a result of the heating step.

### Surface Analysis - AFM & SEM

PCL films prepared as described above were imaged using Atomic Force Microscopy (AFM, Veeco CP II) and Philips XL30 Field Emission Gun Scanning Electron Microscopy (SEM) techniques.

In the case of analysis of films formed using the patterning process, a scanning frequency of approximately 0.4 kHz was used in tapping mode for the AFM imaging. The scanned area was 80x80 µm². Samples were gold coated in a sputter coater (Edwards Ltd.) and mounted onto Aluminium stubs (Agar Scientific Ltd.) prior to SEM imaging and the microscope was operated at 5 kV with a 20 mm working distance.

### Analysis of surface patterning

The photolithography and subsequent HF and KOH etching steps were found to be efficient approaches to produce the silicon master template for the surface patterning of PCL films (Figure 4). The depth of these microgrooves was approximately 5µm and the control material (not etched in KOH) 500 nm. Rather than being upright, the walls of the grooves were truncated V-shape at an angle of 54.74° due to the anisotropic etching fashion of KOH through silicon. Importantly, the patterns on these silicon wafers were transferred with high fidelity to the biocompatible and biodegradable polymers described herein.

### Surface Analysis - XPS

X-ray Photoelectron Spectroscopy (XPS, AXIS Ultra) was used to analyse the chemical and electronic state of the carbon and oxygen elements existing in the PCL film before and after treatment with NaOH.

XPS spectra for a PCL film before and after NaOH treatment showed a reduced peak for the C-O group, which confirms that alkaline hydrolysis has cleaved the ester bond.

### Mechanical Testing

The tensile strength, Young's modulus and maximum strain of PCL were measured, before and after NaOH treatment.

Tensile strength is defined as the maximum amount of tensile stress that a material can be subjected to before failure. Young's modulus is a measurement of stiffness. Maximum strain is measured as the total elongation per unit length of material subject to same applied stress.

The mechanical property of films was measured by Instron 1122 at 23±1°C temperature, 50%±2% relative humidity. The grip distance and crosshead speed were set on 10mm and 50mm/min, respectively. The full load of 0.02KN was used for PCL films and 0.01 KN for PCL/PLA films.

The films were cast on silicon template and were approximately 25x25 mm² in size. The groove on silicon substrates has five different dimensions which illustrated as width-space-depth (µm); 10-10-5, 10-20-5, 20-20-3 and 20-20-5. 10 specimens were prepared for each pattern and their width and thickness were measured by digital micrometer.

The results are set out in the following tables.

**Table 1: Mechanical strength of PCL films, before and after NaOH treatment.**

| **Untreated** | **Modulus (MPa)** | **BRK.STR (MPa)** | **BRK.STN (mm/mm)** | **Width (mm)** | **Thickness (mm)** |
|---|---|---|---|---|---|
| **PCL 10+10+5** | 83.75± 12.47 | 3.18± 0.53 | 3.49± 0.68 | 11.29± 0.29 | 0.054± 0.004 |
| **PCL 10+20+5** | 73.89± 19.71 | 2.86± 0.62 | 2.36± 0.66 | 11.01± 0.24 | 0.052± 0.002 |
| **PCL 20+20+3** | 84.91± 10.67 | 2.71± 0.36 | 2.57± 0.29 | 11.13± 0.29 | 0.053± 0.005 |
| **PCL 20+20+5** | 88.33± 12.29 | 3.09± 0.42 | 2.72± 0.63 | 11.21± 0.42 | 0.054± 0.003 |
| **PCL 20+20+10** | 95.65± 10.03 | 3.34± 0.93 | 2.79± 0.58 | 10.91± 0.42 | 55.56± 0.003 |

| **NaOH treated** | | | | | |
|---|---|---|---|---|---|
| **PCL 10+10+5** | 92.41± 14.21 | 3.81± 1.11 | 3.06± 0.71 | 11.22± 0.20 | 0.052± 0.004 |
| **PCL 10+20+5** | 78.73± 16.06 | 2.83± 0.55 | 2.25± 0.62 | 11.02± 0.37 | 0.054± 0.005 |
| **PCL 20+20+3** | 88.59± 18.27 | 2.69± 0.43 | 2.04± 0.72 | 11.56± 0.35 | 0.050± 0.005 |
| **PCL 20+20+5** | 83.80± 8.30 | 2.98± 0.86 | 2.46± 0.609 | 11.23± 0.48 | 0.055± 0.006 |
| **PCL 20+20+10** | 89.37± 10.53 | 2.78± 0.53 | 2.46± 0.92 | 11.07± 0.28 | 0.055± 0.002 |

**Table 2: Mechanical strength of PCL/PLA films, before and after NaOH treatment.**

| **Untreated** | **Young modulus (MPa)** | **BRK.ST R (MPa)** | **BRK.ST N (mm/mm)** | **Width (mm)** | **Thickness (mm)** |
|---|---|---|---|---|---|
| **PCL/PLA 10-10-5** | 29.878 ± 10.638 | 1.203 ± 0.324 | 2.192 ± 0.567 | 11.802 ± 0.593 | 0.177 ± 0.035 |
| **PCUPLA 10-20-5** | 28.873± 12.036 | 1.458± 0.249 | 2.212± 0.612 | 11.911 ± 0.548 | 0.142± 0.021 |
| **PCUPLA 20-20-3** | 50.37 ± 14.157 | 2.049± 0.671 | 2.387± 0.660 | 12.084 ± 0.717 | 0.118± 0.030 |
| **PCUPLA 20-20-5** | 49.026 ± 14.487 | 1.777± 0.339 | 2,312± 0.528 | 11.841 ± 0.515 | 0.125± 0.024 |
| **PCL/PLA 20-20-10** | 45.012 ± 12.416 | 1.998± 0.481 | 2.399± 0.417 | 11.647 ± 0.763 | 0.116± 0.023 |

| **NAOH treated** | | | | | |
|---|---|---|---|---|---|
| **PCL/PLA 10-10-5** | 22.166± 7.519 | 0.996± 0.178 | 2.606± 0.856 | 12.587 ± 0.584 | 0.111± 0.016 |
| **PCUPLA 10-20-5** | 21.639± 7.653 | 1.074± 0.347 | 2.246± 0.402 | 11.83± 0.641 | 0.092± 0.027 |
| **PCL/PLA 20-20-3** | 21.319± 7.785 | 1.322± 0.435 | 2.324± 0.838 | 12.1± 0.893 | 0.085± 0.028 |
| **PCL/PLA 20-20-5** | 23.786± 8.218 | 1.086± 0.295 | 1.971± 0.469 | 12.289 ± 0.819 | 0.097± 0.025 |
| **PCUPLA 20-20-10** | 28.410± 8.975 | 1.260± 0.351 | 2.192± 0.661 | 11.772 ± 0.895 | 0.081± 0.024 |

The results show that microgrooved PCL and PCUPLA films can be fabricated at micro-thickness and at the same time retain mechanical strength and flexibility.

### Cell Compatibility Analysis

### Cell Source

The NG108-15 cell line was purchased from ECACC (Porton Down, UK). Schwann cells were isolated from neonate rats as previously described [3] and maintained with 63ng/ml glial growth factor (GGF) and 10 µM forskolin mitogen supplemented media.

### Cell culturing and differentiation

NG108-15 cells were maintained in high glucose (4.5g/ml) DMEM (Dulbecco's Modified Eagle's Medium) medium, containing 5% fetal bovine serum, 1% Penicillin/Streptomycin, and supplemented with 1x HAT solution at 37°C in a 5% CO₂ humidified atmosphere. To induce differentiation, NG108-15 cells (1000/cm²) were cultured onto patterned PCL films in medium containing 10% serum and 1% antibiotics for 2 days. On day 3, cell culture medium was replaced with medium containing 1.5% serum, 1% antibiotics and 1 mM dibutyryl-cAMP. Cells were cultured for another 5 days. Schwann cells were cultured at the density of 10⁴/cm² for 6 days before SEM and antibody staining (see below) were conducted.

### SEM imaging of cells

Cells growing on patterned PCL films were prepared for SEM imaging using hexamethyldisilazane (HMDS) chemical drying method[4]. Cells were rinsed twice in PBS and then fixed with 1.5% glutaraldehyde (24.8% stock from TAAB Laboratories) in 0.1 M phosphate buffer (containing NaH₂PO₄.2H₂O 15.6g and Na₂HPO₄ 14.2g in 1000ml dH₂O; pH=7.3) for 30 mins at room temperature. After fixation, cells were rinsed twice with phosphate buffer and dehydrated through a series of increasing concentrations of ethanol solutions (50%, 70%, 90%, and 100%) with 2x5 mins in each solution. Films were then dried in HMDS for 2x5 mins.

Finally, HMDS was removed and the films left overnight in fume cupboard for the fully evaporation of HMDS. Prior to SEM imaging, films were mounted onto an aluminium stub and sputter coated with gold.

### Antibody staining

For immunocytochemical analysis, NG108-15 cells or Schwann cells growing on PCL films were washed gently in PBS and fixed with 4% paraformaldehyde for 30 mins. Following fixation, cells were washed twice in PBS and then permeabilised with 0.2% Triton X-100 for 20 mins. After washing in PBS, a blocking solution of 5% normal goat serum was incubated with the cells for 1 hour at room temperature. The blocking solution was removed and mouse monoclonal anti-neurofilament antibody was used as primary antibody for NG108-15 cells (1:500 dilution; Abcam plc) or rabbit polyclonal S100 for Schwann cells (1:500 dilution; Dako cytomation). Incubation was conducted at 4°C overnight.

Following thorough washing in PBS (3x10mins) to remove non-specifically bound antibodies, goat anti-mouse CY3 secondary antibody for NG108-15 cells (1:200 dilution; Amersham plc) or goat anti- rabbit FITC-conjugated secondary antibody for Schwann cells (1:100 dilution; Vector Labs) was incubated with the cells for 1 hour in dark. Films were then washed again (3x10mins) before they were mounted onto microscope slides.

ProLong® Gold antifade reagent with DAPI (Invitrogen/Gibco) was used to prevent the samples from bleaching and to counter-stain the nuclei of the cells. Pictures were taken using Nikon Eclipse 50i fluorescence microscope.

### Resazurin (Alamar Blue) Assay

Resazurin fluorometric assay was carried out to compare the proliferation rate of NG108-15 cells on micro-grooved polymer films and the porous/non-grooved samples. Briefly, 105 cells (in 100µl suspension) were seeded onto each sample film (25x25mm²) and incubated (37°C, 5% CO2, humidified incubator) for 2 hours before the wells were topped up with 2mls of cell culture medium. To avoid the attachment of cells onto the cell culture plates, ultra low cell attachment plates (Corning) were used in this experiment. On day 1, day 3, day 5 and day 7, cell culture medium was removed and 200µl of Resazurin solution (Resazurin Sodium Salt (Sigma 199303-5G; 0.125% wt/v) in PBS), was added into each well of the 6-well plates together with 2ml of fresh cell culture medium. Samples were incubated (37°C, 5% CO₂, humidified incubator) for 2 hours before the absorbance reading was taken at 540 nm and 630 nm with a standard spectrophotometer. Cell culture medium was used as control.

The results are set out in the table below. Results on day 3, day 5 and day7 showed that NG108-15 cells had a significantly higher proliferation rate on micro-grooved polymer films (p<0.05), including both micro-grooved PCL homopolymer films and the PCL/PLA blend films, than that on the micro-porous scaffolds. In addition, cell proliferation rate was higher on PCL/PLA blend films than that on PCL homopolymer films, regardless of the surface patterning.

**Table 3 - Resazurin assay results**

| **Samples** | **Absorbance (day3)** | **Absorbance (day5)** | **Absorbance (day7)** |
|---|---|---|---|
| PCL/PLA porous | 490.4167 | 571.2083 | 631.1667 |
| PCL porous | 226.8611 | 247.6528 | 285.3333 |
| PCL 10+10+5 | 1769.083 | 2387.986 | 2934.167 |
| PCL 10+20+5 | 2034.972 | 2417.764 | 2974.278 |
| PCL 20+20+5 | 2143.861 | 2564.097 | 2739.611 |
| PCL/PLA 10+10+5 | 2402.583 | 3407.653 | 4393.611 |
| PCL/PLA 10+20+5 | 2414.75 | 3832.653 | 4691.722 |
| PCL/PLA 20+20+5 | 2659.083 | 3927.653 | 4698.278 |

### Cell morphology and alignment

The fraction of cells within ±10° of the patterning direction is the definition adopted to quantify cell alignment.

Although no cell alignment was observed on PCL films with a groove depth of 500 nm, both Schwann cells and NG108-15 cells were found to be successfully aligned along the 5 µm deep grooves. Figure 5 shows the contrast effect of aligned NG108-15 neurites in the patterned area of 10 µm +10 µm against randomly distributed neurites in the unaligned area. Figure 6 shows the SEM image of a differentiated NG108-15 cell with axons aligned along the grooves of a 5 µm +15 µm pattern. Figure 8 shows Schwann cells aligned along the grooves of the patterns with extended processes, which indicated excellent biocompatibility of the material with Schwann cells. Schwann cells were found to attach to the unpatterned, smooth substrate in random directions shown in Figure 7D. The morphology of both cell types varied on patterns of different dimensions. It was observed that NG108-15 cells tended to have more branched processes and the number of neurites also appeared to be higher on wide grooves and spacings i.e. 15 µm and 20 µm, than on patterns of smaller dimensions i.e. 5 µm and 10 µm (Figure 8). The morphology of Schwann cells was also affected by the groove size. It was observed that the processes of Schwann cells tended to be longer on patterns with smaller groove and spacing sizes i.e. 5µm + 5µm, 5µm + 10µm and 10µm + 10µm; when compared with Schwann cells on patterns with bigger dimension (i.e. 15µm + 15µm, 20µm + 20µm). Importantly, contamination of fibroblast cells was reduced on smaller dimension, which was speculated to be resulted from the relatively bigger size of fibroblasts (20-30µm in diameter) than Schwann cells (5-10µm in diameter). The number of fibroblast cells on patterned areas was between 8.7% and 25.8% of that on unpatterned, with the lowest on 5µm + 10µm and the highest on 20µm + 20µm. The reduced adhesion of fibroblast cells onto the patterned PCL films might be advantageous for the axonal regeneration and the proliferation of Schwann cells *in vivo.*

It was observed that both NG108-15 cells and Schwann cells had a tendency to settle in the grooves in all fourteen different patterns, with the average percentage of neural cells in grooves being over 58.2% and Schwann cells 69%. In the case of NG108-15, when the widths of the grooves were larger, more cells were found in the grooves. This effect was clearly seen in patterns of 5 µm + 20 µm, 10 µm + 20 µm, 10 µm + 15 µm and 5 µm + 15 µm; however, when the widths of the spacing were also large (e.g. 20 µm + 20 µm and 20 µm +10 µm), this type of cell response diminished (Figures 9 to 12). On the contrary, patterns with small grooves (5 µm) saw more cells attached on the spacing area, particularly when the widths of spacing were large (e.g. 15 µm + 5 µm and 20 µm + 5 µm).

The design of the pattern with small spacings (5 µm) and large grooves (20 µm) in mask 2 was based on the observation that NG108-15 cells tended to align in the grooves and the chance for the regenerating nerve fibers to grow across the grooves was therefore reduced. This is believed to be unique in our design because other researchers previously adopted the same sizes for both the grooves and the spacings. However, no such effect was found for Schwann cells. This was speculated to be due to the smaller size of Schwann cells, which could align the cells in smaller grooves i.e. 10 µm and 5 µm.

### Results on Mask 2

To make implantable conduits, PCL films with enlarged pattern area (2.7x2.7cm²) were produced from Mask 2. Data obtained from these substrates are shown in the Table below. Differentiated NG108-15 cells were used in this experiment and it was shown that patterns 5 µm +20 µm and 10 µm +10 µm were comparable with each other in performance and 20 µm +20 µm was significantly worse (p<0.05) both in terms of the average neurite length and the alignment rate. Statistics was conducted using Student's T-test: two-samples assuming equal variance.

**Table 4: Average neurite length and neurite alignment rate of differentiated NG108-15 cells on patterned PCL films.**

| **Patterns of Microgrooves** | **5 µm +20 µm** | **10 µm +10 µm** | ***20 µm +20 µm** |
|---|---|---|---|
| Average Neurite Length | 289.8 µm | 279.2 µm | 198.7 µm |
| *Neurite Alignment Rate* | 86.7% | 84.5% | 71.9% |

| | | | |
|---|---|---|---|
| * Performance of pattern 20 µm +20 µm was significantly (p<0.05) less than that of patterns 5 µm +20 µm, and 10 µm +10 µm. | | | |

The results of the present investigation with differentiated NG108-15 cells, showed that both the ridge width and the groove width played important roles in guiding and deciding the orientation of regenerating nerve fibers. In particular the present study, small ridge width (5 µm) combined with big groove width (10 µm, 15 µm and 20 µm) were found to produce neurites that were considerably longer as compared to the ridge and groove widths being the same and the cross-growth of nerve fibres across the substrata was also reduced.

Embodiments of the present invention provide a simple and efficient approach to producing micro-grooves on the surface of biodegradable ultra-thin polymer films, which can be easily rolled up to form implantable nerve conduits. The use of grooves that are wider than the ridges provides enhanced contact guidance of regenerating axons. With the added advantages of the ultra-thin polymer scaffolds, such as excellent handle-ability, low cost and easy availability, these patterned conduits are adapted for peripheral nerve repair.

### Comparison of Smooth, Pitted and Grooved Conduits

In order to further test cell attachment and alignment to the smooth, pitted and grooved (square grooves with vertical walls (SQ); grooves with sloping sidewalls (SL); and grooves with sloping sidewalls and zero ridge width, i.e. V-shaped "ridges" (SLV)) PCUPLA (4:1) films, differentiated adipose stem cells (dASCs) were seeded and allowed to adhere for 24 hours. Adherent cells were fixed with 4% PFA/PBS and stained with Alexa Fluor 488® phalloidin (Invitrogen) and mounted with vectashield containing DAPI (Vector). Cells were imaged using a fluorescent microscope and image-pro plus software. For quantitative analysis of cell attachment 5 separate fields of view were captured for each sample and average cell counts calculated. In addition, cell proliferation/cytotoxicity experiments were carried out using the alamarBlue® assay (abD Serotec) to test cell proliferation of dASCs on grooved and non-grooved PCUPLA (4:1) films. Absorbance readings were taken at 570nm and 600nm using a spectrophotometer and calculated as percentage reduction of alamarBlue®. All experiments were performed in triplicate and the results plotted using GraphPad Prism software. Statistical analysis was carried out using one-way ANOVA followed by Bonferroni's Multiple Comparison Test.

The grooved PCUPLA (4:1) surfaces were prepared as described above. A silicon substrate having grooves with width-space-depth dimensions of 10 µm -10 µm -5 µm was used to form the PCUPLA (4:1) surfaces. This silicon substrate used to form the SQ-grooved PCUPLA (4:1) surface had vertical side walls, resulting in a SQ-grooved PCUPLA (4:1) surface having grooves with width-space-depth dimensions of 10 µm -10 µm -5 µm. The silicon substrate used to form the SL-grooved PCUPLA (4:1) surface had side walls at an angle of 30° to 50° to the vertical, resulting in a SL-grooved PCUPLA (4:1) surface having grooves of width 15 µm to 20 µm, spacing of 3 µm to 6 µm and depth of 5 µm. The silicon substrate used to form the SLV-grooved PCUPLA (4:1) surface had side walls at an angle of 30° to 50° to the vertical, resulting in a SLV-grooved PCUPLA (4:1) surface having grooves of width 15 µm to 20 µm, spacing of 0 µm (V-shaped ridges) and depth of 5 µm. Of course, other width-space-depth dimensions may be used, as discussed above.

A first observation is that statistically significant increases in cell attachment are achieved for the pitted PCUPLA (4:1) surface when compared to the smooth PCUPLA (4:1) surface (Figure 13). Statistically significant increases in cell attachment were also observed in SL-grooved and SLV-grooved PCUPLA (4:1) film surfaces when compared to the smooth surface. An increase in cell attachment of the SQ-grooved surface were also observed (Figure 13). Representative images illustrated these differences in cell attachment whilst further demonstrating increased cell alignment to a spindle-like morphology for dASCs attached to the grooved PCUPLA (4:1) film surfaces. This alignment was found to be most noticeable on the SLV-grooved and SL-grooved samples but also on the SQ-grooved samples when compared with the pitted samples (Figure 14). Cell attachment data (Figure 15) for the grooved PCUPLA samples was corroborated by the proliferation data that demonstrated statistically significant increases in proliferation for the SL-grooved (p<0.05) and SLV-grooved (p<0.01) samples when compared with the non-grooved control.

Taken together these results demonstrate that topographical modifications to PCUPLA surfaces, especially the sloping wall variants SL and SLV, significantly enhance dASC attachment and proliferation. Furthermore these modifications result in the highly bordered alignment of dASCs to a spindle shaped schwann cell-like morphology that resemble longitudinally aligned bands of Bungner found in damaged nerves following wallerian degeneration that provide essential pathways for guided axonal growth. Thus these topographical features will improve axonal guidance and enhance nerve regeneration in PCUPLA nerve conduits.

### REFERENCES

A number of publications are cited herein in order to more fully describe and disclose the invention and the state of the art to which the invention pertains. Full citations for these references are provided below.
[1] Kalbermatten, D.F. et al., "Fibrin matrix for suspension regenerative cells in an artificial nerve conduit", Journal of Plastic, Reconstructive & Aesthetic Surgery (2008), Volume 61, Issue 6, Pages 669-675.
[2] Rasband, W.S., Image J, U. S. National Institutes of Health, Bethesda, Maryland, USA, http://rsb.info.nih.gov/ij/, 1997-2008.
[3] Caddick, J. et al., "Phenotypic and functional characteristics of mesenchymal stem cells differentiated along a Schwann cell lineage", Glia 54 (2006), pp. 840-849.
[4] J.L. Nation, A new method using hexamethyldisilazane for preparation of soft insect tissues for scanning electron microscopy. Stain Tech. 58(1983)347-351.

## Claims

1. A peripheral nerve growth scaffold that is a tubular conduit including poly-ε-caprolactone (PCL), the tubular conduit having a tubular conduit wall surrounding and defining a luminal space, said tubular conduit wall having a thickness in the range 10µm to 300µm; the tubular conduit having microgrooves on its luminal surface, wherein the microgrooves comprise at least one sloping sidewall, the or each side wall forming an angle to the vertical of at least 20°.

2. A peripheral nerve growth scaffold according to claim 1, wherein the or each side wall forms an angle to the vertical of at least 40°

3. A peripheral nerve growth scaffold according to claim 1 or claim 2 wherein the microgrooves have a truncated V-shape cross-section.

4. A peripheral nerve growth scaffold according to any one of claims 1 to 3 wherein the outer surface of the tubular conduit is not provided with microgrooves.

5. A peripheral nerve growth scaffold according to any one of the preceding claims, wherein the tubular conduit wall is substantially free of pores extending through the thickness of the wall.

6. A peripheral nerve growth scaffold according to any one of the preceding claims, wherein the scaffold comprises at least 60wt% PCL based on the total weight of the conduit and the scaffold also includes polylactic acid and (PLA), the PLA being present as a PCL-PLA blend, wherein the weight ratio of PCL:PLA is in the range 6:1 to 2:1.

7. A peripheral nerve growth scaffold according to any one of the preceding claims, wherein the microgrooves have a width:spacing ratio in the range 1.5:1 to 8:1, preferably in the range 2.5:1 to 5:1.

8. A peripheral nerve growth scaffold according to any one of the preceding claims, wherein the microgrooves have a width in the range 10µm to 40µm, preferably in the range 15µm to 25µm.

9. A peripheral nerve growth scaffold according to any one of the preceding claims, wherein the microgrooves have a spacing in the range 5µm to 15µm.

10. A peripheral nerve growth scaffold according to any one of the preceding claims, wherein the microgrooves have a depth in the range 2µm to 10µm.

11. A peripheral nerve growth scaffold according to any one of the preceding claims, wherein the thickness of the tubular conduit wall is in the range 20µm to 80µm.

12. A peripheral nerve growth scaffold according to any one of the preceding claims, wherein the scaffold has a length in the range 5mm to 50mm and a diameter in the range 1 to 5mm.

13. A peripheral nerve growth scaffold according to any one of the preceding claims, wherein the scaffold is made from a film comprising PCL wherein the film has been cast onto a patterned template so as to form the microgrooves and opposite edges of the film are joined together by heat sealing to form the scaffold.

14. A method of making a peripheral nerve growth scaffold as defined in any one of claims 1 to 13, the method comprising the step of:
i) solvent casting a film including PCL directly onto a patterned template, and allowing the solvent to evaporate; and
ii) joining opposite edges of the film together by heat sealing to form the scaffold.

## Patentansprüche

1. Gerüst für peripheres Nervenwachstum, das einen rohrförmigen Kanal umfasst, der Poly-ε-Caprolacton (PCL) umfasst, wobei der rohrförmige Kanal eine rohrförmige Kanalwand aufweist, die einen Lumenraum umgibt und definiert, wobei die rohrförmige Kanalwand eine Dicke im Bereich 10 µm bis 300 µm aufweist; wobei der rohrförmige Kanal Mikrorillen auf seiner Lumenoberfläche aufweist, wobei die Mikrorillen zumindest eine geneigte Seitenwand umfassen, wobei die oder jede Seitenwand einen Winkel zur Vertikalen von zumindest 20 ° bildet.

2. Gerüst für peripheres Nervenwachstum nach Anspruch 1, wobei die oder jede Seitenwand einen Winkel zur Vertikalen von zumindest 40 ° bildet.

3. Gerüst für peripheres Nervenwachstum nach Anspruch 1 oder 2, wobei die Mikrorillen einen abgeschnittenen V-förmigen Querschnitt aufweisen.

4. Gerüst für peripheres Nervenwachstum nach einem der Ansprüche 1 bis 3, wobei die Außenfläche des rohrförmigen Kanals nicht mit Mikrorillen bereitgestellt ist.

5. Gerüst für peripheres Nervenwachstum nach einem der vorhergehenden Ansprüche, wobei die rohrförmige Kanalwand im Wesentlichen frei von Poren ist, die sich durch die Dicke der Wand hindurch erstrecken.

6. Gerüst für peripheres Nervenwachstum nach einem der vorhergehenden Ansprüche, wobei das Gerüst zumindest 60 Gew.-% PCL basierend auf dem Gesamtgewicht der Leitung umfasst, und das Gerüst ferner Polymilchsäure und (PLA) umfasst, wobei das PLA als PCL-PLA-Mischung vorliegt, wobei das Gewichtsverhältnis von PCL:PLA im Bereich 6:1 bis 2:1 liegt.

7. Gerüst für peripheres Nervenwachstum nach einem der vorhergehenden Ansprüche, wobei die Mikrorillen ein Verhältnis Breite:Beabstandung im Bereich 1,5:1 bis 9:1 aufweisen, vorzugsweise im Bereich 2,5:1 bis 5:1.

8. Gerüst für peripheres Nervenwachstum nach einem der vorhergehenden Ansprüche, wobei die Mikrorillen eine Breite im Bereich von 10 µm bis 40 µm, vorzugsweise im Bereich 15 µm bis 25 µm aufweisen.

9. Gerüst für peripheres Nervenwachstum nach einem der vorhergehenden Ansprüche, wobei die Mikrorillen eine Beabstandung im Bereich von 5 µm bis 15 µm aufweisen.

10. Gerüst für peripheres Nervenwachstum nach einem der vorhergehenden Ansprüche, wobei die Mikrorillen eine Tiefe im Bereich von 2 µm bis 10 µm aufweisen.

11. Gerüst für peripheres Nervenwachstum nach einem der vorhergehenden Ansprüche, wobei die Dicke der rohrförmigen Kanalwand im Bereich von 20 µm bis 80 µm liegt.

12. Gerüst für peripheres Nervenwachstum nach einem der vorhergehenden Ansprüche, wobei das Gerüst eine Länge im Bereich von 5 mm bis 50 mm und einen Durchmesser im Bereich von 1 bis 5 mm aufweist.

13. Gerüst für peripheres Nervenwachstum nach einem der vorhergehenden Ansprüche, wobei das Gerüst aus einem Film hergestellt ist, das PCL umfasst, wobei der Film auf eine strukturierte Schablone gegossen wurde, um die Mikrorillen auszubilden, und gegenüberliegende Kanten des Films durch Heißsiegeln miteinander verbunden werden, um das Gerüst auszubilden.

14. Verfahren zur Herstellung eines Gerüsts für peripheres Nervenwachstum nach der Definition eines der Ansprüche 1 bis 13, wobei das Verfahren folgenden Schritt umfasst:
i) Solvent-Casting-Gießen eines Films, der PCL umfasst, direkt auf eine strukturierte Schablone, und Verdampfenlassen des Lösemittels; und
ii) Verbinden von gegenüberliegenden Kanten des Films durch Heißsiegeln, um das Gerüst auszubilden.

## Revendications

1. Echafaudage de croissance de nerf périphérique, qui est un conduit tubulaire incluant le poly-ε-caprolactone (PCL), le conduit tubulaire ayant une paroi de conduit tubulaire et définissant un espace luminal, ladite paroi de conduit tubulaire ayant une épaisseur dans la plage de 10 µm à 300 µm ; le conduit tubulaire ayant des micro-rainures sur sa face luminale, où les micro-rainures comprennent au moins une paroi latérale inclinée, la ou chaque paroi latérale formant un angle à la verticale d'au moins 20°.

2. Echafaudage de croissance de nerf périphérique selon la revendication 1, dans lequel la ou chaque paroi latérale forme un angle à la verticale d'au moins 40°.

3. Echafaudage de croissance de nerf périphérique selon la revendication 1 ou la revendication 2, dans lequel les micro-rainures ont une section transversale en V tronquée.

4. Echafaudage de croissance de nerf périphérique selon l'une quelconque des revendications 1 à 3, dans lequel la surface extérieure du conduit tubulaire n'est pas pourvue de micro-rainures.

5. Echafaudage de croissance de nerf périphérique selon l'une quelconque des revendications précédentes, dans lequel la paroi de conduit tubulaire est sensiblement exemple de pores s'étendant à travers l'épaisseur de la paroi.

6. Echafaudage de croissance de nerf périphérique selon l'une quelconque des revendications précédentes, dans lequel l'échafaudage comprend au moins 60 % en poids de PCL basé sur le poids total du conduit, et l'échafaudage comprend également de l'acide poly-lactique et (PLA), le PLA étant présent comme mélange de PCL-PLA, où le rapport pondéral de PCL:PLA est dans la plage de 6:1 à 2:1.

7. Echafaudage de croissance de nerf périphérique selon l'une quelconque des revendications précédentes, dans lequel les micro-rainures ont un rapport de largueur: espacement dans la plage de 1,5:1 à 8:1, de préférence dans la plage de 2,5:1 à 5:1.

8. Echafaudage de croissance de nerf périphérique selon l'une quelconque des revendications précédentes, dans lequel les micro-rainures ont une largueur dans la plage de 10 µm à 40 µm, de préférence dans la plage de 15 µm à 25 µm.

9. Echafaudage de croissance de nerf périphérique selon l'une quelconque des revendications précédentes, dans lequel les micro-rainures ont un espacement dans la plage de 5 µm à 15 µm.

10. Echafaudage de croissance de nerf périphérique selon l'une quelconque des revendications précédentes, dans lequel les micro-rainures ont une profondeur dans la plage de 2 µm à 10 µm.

11. Echafaudage de croissance de nerf périphérique selon l'une quelconque des revendications précédentes, dans lequel l'épaisseur de la paroi de conduit tubulaire est dans la plage de 20 µm à 80 µm.

12. Echafaudage de croissance de nerf périphérique selon l'une quelconque des revendications précédentes, dans lequel l'échafaudage a une longueur dans la plage de 5 mm à 50 mm et un diamètre dans la plage de 1 à 5 mm.

13. Echafaudage de croissance de nerf périphérique selon l'une quelconque des revendications précédentes, dans lequel l'échafaudage est réalisé à partir d'un film comprenant du PCL, où le film a été coulé sur un gabarit à motif de manière à former les micro-rainures, et des bords opposés du film sont joints ensemble par thermo-soudage pour former l'échafaudage.

14. Procédé de réalisation d'un échafaudage de croissance de nerf périphérique tel que défini dans l'une quelconque des revendications 1 à 3, le procédé comprenant l'étape de :
i) couler avec un solvant un film incluant du PCL directement sur un gabarit à motif, et permettre au solvant de s'évaporer ; et
ii) joindre des bords opposés du film ensemble par thermo-soudage pour former l'échafaudage.
